# EUROPEAN PATENT APPLICATION

(11) **EP 0 957 108 A1**
(43) Date of publication of application: **17.11.1999**
(21) Application number: 98250166.0
(22) Date of filing: 14.05.1998
(51) Int. Cl.: C07J 9/00, A61K 31/575, C07J 41/00, C07J 53/00

(54) **Unsaturated cholestane derivatives, pharmaceutical compositions comprising them and use of these derivatives for the preparation of meiosis regulating medicaments**

(71) Applicant: SCHERING AKTIENGESELLSCHAFT, 13342 Berlin (DE)
(72) Inventor: Blume, Thorsten, Dr., 13467 Berlin (DE); Esperling, Peter, 12107 Berlin (DE); Kuhnke, Joachim, 14089 Berlin (DE); Hegele-Hartung, Christa, 45470 Mülheim/Ruhr (DE); Lessl, Monika, 13467 Berlin (DE)

(57) **Abstract**

The present invention relates to pharmaceutically active unsaturated cholestane derivatives, to pharmaceutical compositions comprising them as active substances and to the use of these novel compounds for the preparation of medicaments. More particularly it has been found that the unsaturated cholestane derivatives of the invention can be used for regulating meiosis.

## Description

The present invention relates to pharmaceutically active unsaturated cholestane derivatives, to pharmaceutical compositions comprising them as active substances and to the use of these novel compounds for the preparation of medicaments. More particularly it has been found that the unsaturated cholestane derivatives of the invention can be used for regulating meiosis.

Meiosis is the unique and ultimate event of germ cells on which sexual reproduction is based. Meiosis comprises two meiotic divisions. During the first division, exchange between maternal and paternal genes take place before the pairs of chromosomes are separated into the two daughter cells. These contain only half the number (1n) of chromosomes and 2c DNA. The second meiotic division proceeds without a DNA synthesis. This division therefore results in the formation of the haploid germ cells with only 1c DNA.

The meiotic events are similar in the male and female germ cells, but the time schedule and the differentiation processes which lead to ova and to spermatozoa differ profoundly. All female germ cells enter the prophase of the first meiotic division early in life, often before birth, but all are arrested as oocytes later in the prophase (dictyate state) until ovulation after puberty. Thus, from early life the female has a stock of oocytes which is drawn upon until the stock is exhausted. Meiosis in females is not completed until after fertilization, and results in only one ovum and two abortive polar bodies per germ cell. In contrast, only some of the male germ cells enter meiosis from puberty and leave a stem population of germ cells throughout life. Once initiated, meiosis in the male cell proceeds without significant delay and produces 4 spermatozoa.

Only little is known about the mechanism which control the initiation of meiosis in the male and in the female. In the oocyte, new studies indicate that follicular purines, hypoxanthine or adenosine, could be responsible for meiotic arrest (Downs, S.M. *et al. Dev Biol* **82** (1985) 454-458: Epplg. J. J. *et al Dev Biol* **119** (1986) 313-321; and Downs, S.M. *Mol Reprod Dev* **35** (1993)82-94). The presence of a diffusible meiosis regulating substance was first described by Byskov *et al.* in a culture system of fetal mouse gonads (Byskov, A. G. *et al. Dev Biol* **52** (1976) 193-200). A meiosis activating substance (MAS) was secreted by the fetal mouse ovary in which meiosis was ongoing, and a meiosis preventing substance (MPS) was released from the morphologically differentiated testis with resting, non-meiotic germ cells. It was suggested that the relative concentrations of MAS and MPS regulated the beginning, arrest and resumption of meiosis in the male and in the female germ cells (Byskov, A.G. et al. in *The Physiology of Reproduction* (eds. Knobil. E. and Neill, J. D., Raven Press, New York (1994)). Clearly, if meiosis can be regulated, reproduction can be controlled. A recent article (Byskov, A. G. et al. *Nature* **374** (1995), 559-562) describes the isolation from bull testes and from human follicular fluid of certain sterols that activate oocyte meiosis. Unfortunately, these sterols are rather labile and utilization of the interesting finding would thus be greatly facilitated if more stable meiosis activating compounds were available.

Compounds being known to stimulate the meiosis and being different from the compounds claimed in the present patent application are described in WO 96/27658.

It is a purpose of the present invention to provide novel compounds for the treatment of infertility in females and males, particularly in humans via activation of meiosis.

It is a further purpose of the present invention to provide novel compounds useful as contraceptives in females and males, particularly in humans via inhibition of meiosis.

According to the present invention there are provided novel, stable compounds with interesting pharmacological properties. In particular, the compounds described in this patent application are useful for the regulation of meiosis in oocytes and in male germ cells.

The present invention relates to unsaturated cholestane derivatives of the general formula I 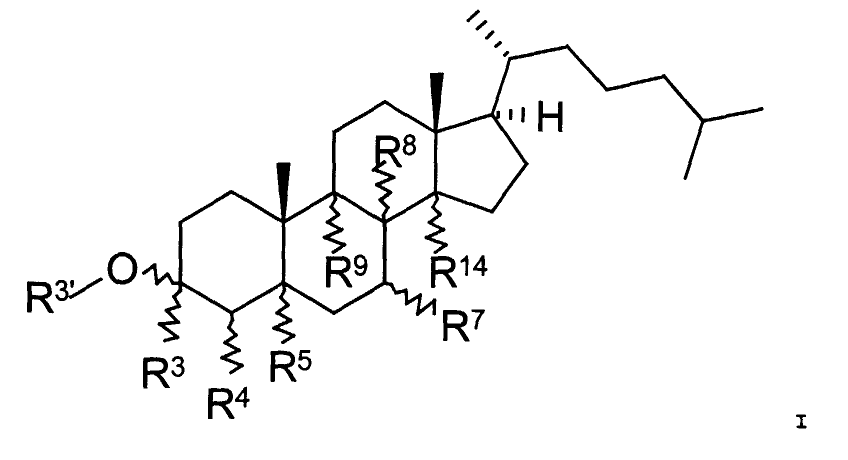 wherein
- R³: designates a hydrogen atom, a C₁-C₆-alkyl group, a C₁-C₆-perfluoroalkyl group or together with R^{3'} an additional bond,
- R^{3'}: designates a hydrogen atom or together with R³ an additional bond,
- R⁴: designates a hydrogen atom or together with R⁵ a methano bridge or together with R⁵ an additional bond,
- R⁵: designates a C₁-C₆-alkyl group, a cyano group, a hydroxymethyl group, a carbaldehyde, an oxime derived from a carbaldehyde, a carboxylic acid, a primary or secondary amide derived from a carboxylic acid, an ester with a C₁-C₆-alcohol group or together with R⁴ a methano bridge or together with R⁴ an additional bond,
- R⁷: designates together with R⁸ an additional bond or a hydrogen atom, if R⁸ and R⁹ or R⁸ and R¹⁴ stand together for an additional bond,
- R⁸: designates together with R⁷ or with R⁹ or with R¹⁴ an additional bond,
- R⁹: designates together with R⁸ an additional bond or a hydrogen atom, if R⁷ and R⁸ or R⁸ and R¹⁴ stand together for an additional bond,
- R¹⁴: designates together with R⁸ an additional bond or a hydrogen atom, if R⁷ and R⁸ or R⁸ and R⁹ stand together for an additional bond,
or esters thereof, with the proviso that the following compounds, which are known from the literature, are excluded:
cholesta-4,7-dien-3-one, cholesta-4,8-dien-3-one, cholesta-4,8(14)-dien-3-one, cholesta-4,7-dien-3α-ol, cholesta-4,7-dien-3β-ol, 5-cyano-5β-cholest-7-en-3-one, 5-cyano-5β-cholest-7-en-3β-ol, 5-methyl-5β-cholest-7-en-3-one, 5-methyl-5β-cholest-7-en-3α-ol, 5-methyl-5β-cholest-7-en-3β-ol.

The compounds of the general formula I have a number of chiral centres in the molecule and thus exist in several isomeric forms. All these isomeric forms and mixtures thereof are within the scope of the invention.

Preferred compounds of formula I are such wherein R³ designates a hydrogen atom, a C₁-C₆-perfluoroalkyl group or together with R^{3'} an additional bond. Especially preferred are compounds of formula I wherein R³ is a C₁-C₃-perfluoroalkyl group, preferably a trifluoromethyl group.

Other preferred compounds of formula I are such wherein R⁵ designates a C₁-C₃-alkyl group, preferably a methyl group, a cyano group, a hydroxymethyl group, or together with R⁴ a methano bridge or together with R⁴ an additional bond.

In another embodiment, the present invention relates to esters of compounds of the general formula I. Such esters are formally derived by esterification of one or more hydroxylic groups of a compound of formula I with an acid which can for example be selected from the group of acids comprising succinic acid and other aliphatic dicarboxylic acids, nicotinic acid, isonicotinic acid, ethylcarbonic acid, phosphoric acid, sulphonic acid, sulphamic acid, benzoic acid, acetic acid, propionic acid and other aliphatic monocarboxylic acids.

As used in the present description and claims, an alkyl group - when used alone or in combinations - may be a straight or branched alkyl group. The expression C₁-C₃ alkyl designates an alkyl group having from one to three carbon atoms: preferred examples are methyl, ethyl and propyl, more preferred methyl and ethyl. Similarly, the expression C₁-C₆ alkyl designates an alkyl group having from one to six carbon atoms; preferred examples are methyl, ethyl, propyl, isopropyl, butyl, tert-butyl, pentyl and hexyl, more preferred methyl, ethyl, propyl, isopropyl, butyl and tert-butyl, still more preferred methyl and ethyl.

Especially preferred compounds of formula I of the present invention are the following:
cholesta-4,8-dien-3β-ol,
cholesta-4,8-dien-3α-ol,
cholesta-4,8(14)-dien-3β-ol,
cholesta-4,8(14)-dien-3α-ol,
5-cyano-5α-cholest-7-en-3α-ol,
5-cyano-5α-cholest-7-en-3β-ol,
5-cyano-5β-cholest-7-en-3α-ol,
5-cyano-5α-cholest-8-en-3α-ol,
5-cyano-5α-cholest-8-en-3β-ol,
5-cyano-5β-cholest-8-en-3α-ol,
5-cyano-5β-cholest-8-en-3β-ol,
5-cyano-5α-cholest-8(14)-en-3α-ol,
5-cyano-5α-cholest-8(14)-en-3β-ol,
5-cyano-5β-cholest-8(14)-en-3α-ol,
5-cyano-5β-cholest-8(14)-en-3β-ol,
3',4α-dihydrocyclopropa[4,5]-5β-cholest-7-en-3β-ol,
3',4β-dihydrocyclopropa[4,5]-5α-chloest-7-en-3α-ol,
3',4α-dihydrocyclopropa[4,5]-5β-cholest-8-en-3β-ol,
3',4β-dihydrocyclopropa[4,5]-5α-cholest-8-en-3α-ol,
3',4α-dihydrocyclopropa[4,5]-5β-cholest-8(14)-en-3β-ol,
3',4β-dihydrocyclopropa[4,5]-5α-cholest-8(14)-en-3α-ol,
5-(hydroxymethyl)-5α-cholest-7-en-3β-ol,
5-(hydroxymethyl)-5β-cholest-7-en-3α-ol,
5-(hydroxymethyl)-5α-cholest-8-en-3β-ol,
5-(hydroxymethyl)-5β-cholest-8-en-3α-ol,
5-(hydroxymethyl)-5α-cholest-8(14)-en-3β-ol,
5-(hydroxymethyl)-5β-cholest-8(14)-en-3α-ol,
5-methyl-5β-cholest-8-en-3-one,
5-methyl-5β-cholest-8-en-3β-ol,
5-methyl-5β-cholest-8-en-3α-ol,
5-methyl-5β-cholest-8(14)-en-3-one,
5-methyl-5β-cholest-8(14)-en-3β-ol,
5-methyl-5β-cholest-8(14)-en-3α-ol,
3α-(trifluoromethyl)-cholesta-4,7-dien-3β-ol,
3β-(trifluoromethyl)-cholesta-4,7-dien-3α-ol,
3α-(trifluoromethyl)-cholesta-4,8-dien-3β-ol,
3β-(trifluoromethyl)-cholesta-4,8-dien-3α-ol,
3α-(trifluoromethyl)-cholesta-4,8(14)-dien-3β-ol,
3β-(trifluoromethyl)-cholesta-4,8(14)-dien-3α-ol.

The compounds of the general formula I according to the invention can be synthesized analogously with the preparation of known compounds. Hence, synthesis of the compounds of formula I can follow the well established synthetic pathways described in the comprehensive sterol and steroid literature. The following books can be used as the key source in the synthesis: L.F. Fieser & M. Fieser: Steroids: Reinhold Publishing Corporation, NY 1959; Rood's Chemistry of Carbon Compounds (editor: S. Coffrey): Elsevier Publishing Company, 1971; and especially Dictionary of Steriods (editors: R.A. Hill; D.N. Kirk; H.L.J. Makin and G.M. Murphy): Chapman & Hall. The last one contains an extensive list of citations to the original papers covering the period up to 1990. All these books including the last mentioned citations are incorporated by reference.

Particularly, the compounds of the present invention are synthesized according to the following general procedures:
Cholesta-5,8-dien-3β-ol **1**, which is synthesized as in the literature described [*J*. *Lip.Res*. **37**, 1529, (1996)], can be oxidized in an Oppenauer reaction to give cholesta-4,8-dien-3-one **2** (scheme 1). In this reaction the sterol is treated with a ketone like acetone, quinone or cyclohexanone in the presence of aluminum isopropoxide or aluminum tert-butoxide [e.g. *J. Chem. Soc. Perkin I* 2667 (1994)]. The sterol can also be oxidized with pyridinium dichromate [vgl. *Synth. Commun.* **20**, 1167 (1990)]. The same oxidation reaction can be carried out with cholesta-5,8(14)-dien-3β-ol, which is also synthesized as in the literature described [*J*. *Lip.Res*. **37**, 1529, (1996)] to give cholesta-5,8(14)-dien-3-one. For this ketone a laborious synthesis is described in the literature [*Bull. Soc. Chim*. *Fr*. 2037, (1971)]. Cholesta-4,7-dien-3-one is synthesized according to literature procedures [*Liebigs Ann. Chem.* **542**, 218, (1939)] (in scheme 1 the series with the Δ-8-double bond are shown as an example, analogous reactions have to be performed in the Δ-7- and Δ-8(14)-series).

In the following only the syntheses in the Δ-8-series are described. The derivatives in the Δ-7 and Δ-8(14)-series can be synthesized by a skilled artisan from the corresponding starting materials in the same way.

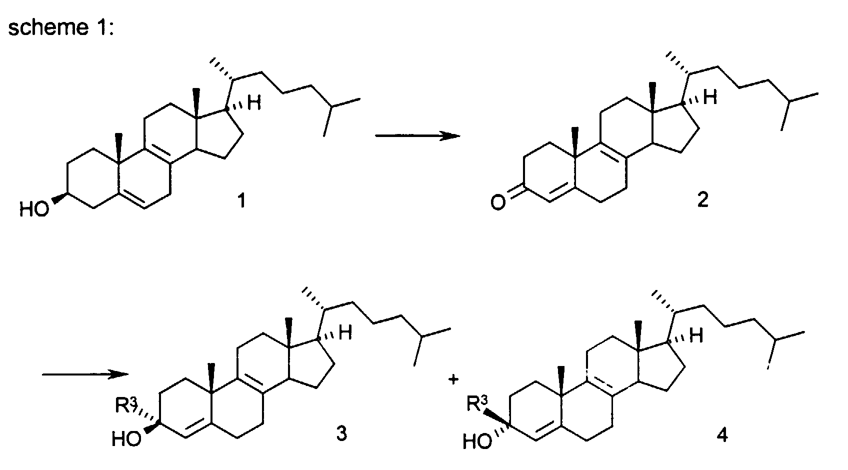

Enone **2** can be treated with different C₁-C₆-Grignard reagents to give two diastereomeric alcohols **3** and **4** (with R³= C₁-C₆-alkyl), which are easily separated by column chromatography [e.g. *J. Med. Chem*. **40,** 61, (1997)].

Cholesta-4,8-dien-3-one **2** can be reduced according to well known literature procedures. Lithium aluminum hydride, sodium borohydride and diisobutylaluminum hydride are especially useful [e.g. *Liebigs Ann. Chem.* **542**, 218, (1939)]. Two diastereomeric alcohols of the formulae **3** and **4** (with R³= hydrogen) can be obtained and readily separated by column chromatography.

For the introduction of perfluoroalkyl substituents in position 3 cholesta-4,8-dien-3-one **2** can be treated with perfluoroalkyl-trialkylsilanes in the presence of fluoride sources like tetrabutyl-ammonium fluoride or caesium flouride. The trifluoromethyl group is preferentially introduced with reagents like trimethylsilyl-trifluoromethane or triethylsilyl-trifluoromethane [*J. Org. Chem*.**56**, 984, (1991); *J. Org. Chem.* **54**, 2873, (1989)].

Again two diastereomeric alcohols of the formulae **3** and **4** (with R³= perfluoroalkyl, preferentially: trifluoromethyl) can be obtained and readily separated by column chromatography.

The cyanoketones **5** and **6** are available starting from cholesta-4,8-dien-3-one **2** via a conjugate addition of cyanide (scheme 2). Different reagents like diethylaluminum cyanide [*J. Org. Chem.* **59**, 2766 (1994)] and some alkali and earth alkali metal cyanides [*Tetrahedron Lett.* **28**, 4189, (1987); *Can. J. Chem.* **59** 1641 (1981)] can be used in this reaction.

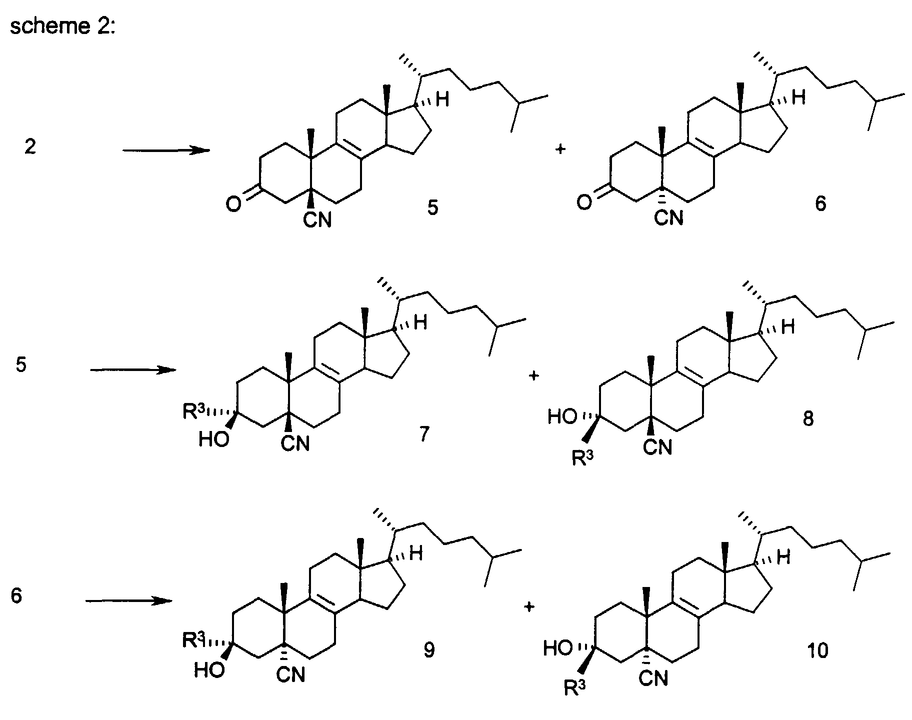

The readily separated cyanoketones **5** and **6** can be reduced according to well known literature procedures. Lithium aluminum hydride, sodium borohydride und diisobutylaluminum hydride are used preferentially [e.g. *Aust. J. Chem.* **35**, 629 (1982)]. In the reduction reactions two diastereomeric alcohols **7** and **8** (R³= H) respectively **9** and **10** (R³= H) are obtained.

The cyanoketones of the formulae **5** and **6** can also be treated with Grignard reagents [e.g. *Chem Pharm Bull.* **9**, 854 (1961)] to give two diastereomeric tertiary alcohols **7** and **8** or **9** and **10** (with R³=C₁-C₆-alkyl) respectively.

The hydroxymethyl derivatives of the formulae **12** and **14** can be synthesized in two step sequences from the cyanoalcohols **8** and **9** respectively (scheme 3). First the cyano group can be reduced with an electrophilic reducing agent like diisobutylaluminum hydride to give the corresponding imines, which are hydrolyzed in situ to the carbaldehydes **11** and **13**. In the second step the carbaldehydes can be further reduced with well known reducing agents like lithium aluminum hydride, sodium borohydnde or diisobutylaluminum hydride to the desired hydroxymethyl derivatives [e.g. *J. Med. Chem.* **39**, 5092, (1996)].

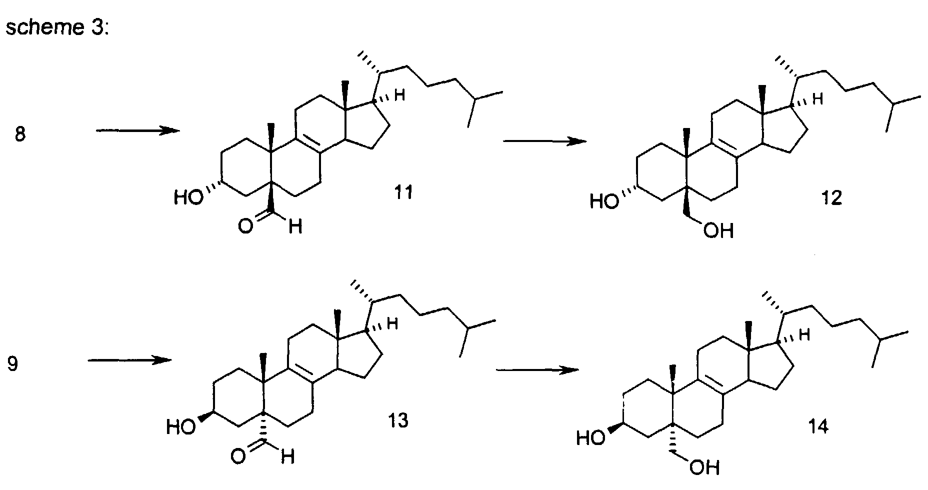

The methano derivatives of the formulae **15** and **16** can be synthesized form the allylic alcohols **3** and **4** respectively (scheme 4). Different variations of the Simmons-Smith reaction can be employed. As reagents diiodomethane in the presence of zinc/copper-couple [e.g. *J. Org. Chem.* **31**, 3869, (1966); *J. Med. Chem.* **39**, 4218 (1996)] as well as chloroiodomethane in the presence of a a diethylzinc solution [*J. Am. Chem. Soc.* **108**, 6343 (1986)] can be used in this cyclopropanation reaction.

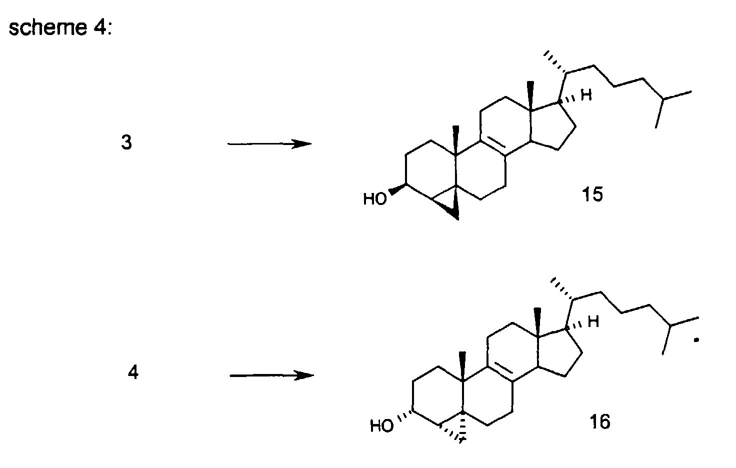

5β-Methyl-cholest-8-en-3-one **17** can be synthesized from cholesta-4,8-dien-3-one **2** via a conjugate addition reaction (scheme 5). The methyl group is introduced either with lithium dimethylcuprate [e.g. *Aust. J. Chem.* **35**, 629, (1982)] or with methylgrignard compounds or trimethylaluminum in the presence of nickel catalysts [e.g. *Tetrahedron Lett.* **35,** 6075, (1994); Synthesis **3**, 317, (1995)].

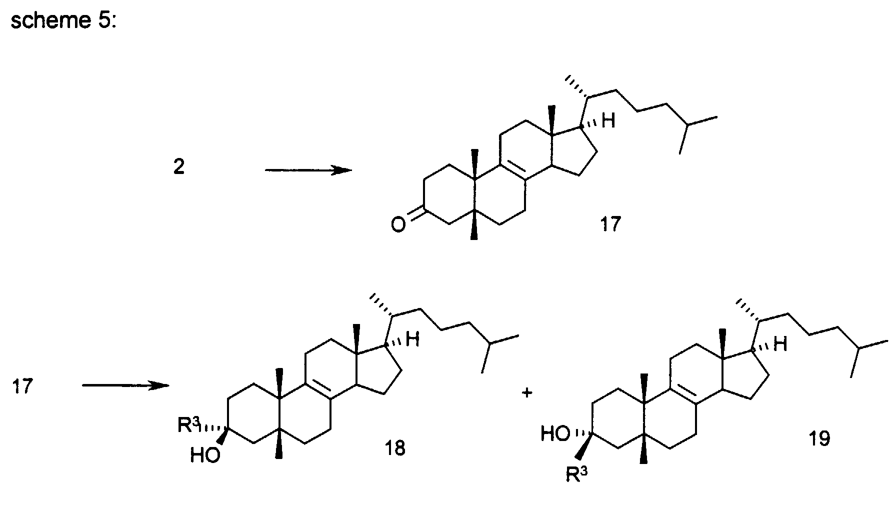

Subsequent reduction of 5β-methyl-ketone **17** is achieved with different well known reducing agents like lithium aluminum hydride, sodium borohydride and diisobutylaluminum hydride [e.g. *Aust. J. Chem*. **35**, 629, (1982)]. Two diastereomeric alcohols **18** and **19** (R³= H) can be obtained and readily separated by column chromatography.
If 5β-methyl-ketone **17** is treated with Grignard reagents two diastereomeric tertiary alcohols (**18** and **19**; mit R³= C₁-C₆-alkyl) are obtained, which are easily separated by column chromatography.

A further object of the present invention are pharmaceutical compositions comprising one or more compounds of the general formula I as active substances. The compositions may further comprise pharmaceutically acceptable excipients well known in the art like carriers, diluents, absorption enhancers, preservatives, buffers, agents for adjusting the osmotic pressure, tablet disintegrating agents and other ingredients which are conventionally used in the art. Examples of solid carriers are magnesium carbonate, magnesium stearate, dextrin, lactose, sugar, talc, gelatin, pectin, tragacanth, methylcellulose, sodium carboxymethyl cellulose, low melting waxes and cacao butter.

Liquid compositions include sterile solutions, suspensions and emulsions. Such liquid compositions may be suitable for injection or for use in connection with *ex vivo* and *in vitro* fertilization. The liquid compositions may contain other ingredients which are conventionally used in the art, some of which are mentioned in the list above. Further, a composition for transdermal administration of a compound of this invention may be provided in the form of a patch and a composition for nasal administraton may be provided in the form of a nasal spray in liquid or powder form. The dose of a compound of the invention to be used will be determined by a physician and will depend among several factors on the particular compound employed, on the route of administration and on the purpose of the use.
In general, the compositions of the invention are prepared by intimately bringing into association the active compound with the liquid or solid auxiliary ingredients and then, if necessary, shaping the product into the desired formulation.

In a further object the present invention concerns the use of the compounds of the general formula I for relieving infertility in females and males, particularly in mammals, more particularly in humans.

In a further object of the present invention the compounds of the general formula I are useful as contraceptives in females and males, particularly in mammals, more particularly in humans.

The compounds of the present invention influence the meiosis in oocytes as well as in male germ cells. The existence of a meiosis inducing substance in nature has been known for some time. However, until recently the identity of the meiosis inducing substance or substances was unknown.

The prospects of being able to influence the meiosis are several. According to a preferred embodiment of the present invention, a compound of the general formula I can be used to stimulate the meiosis. According to another preferred embodiment of the present invention, a compound of the general formula I can be used to stimulate the meiosis in humans. Thus, the compounds of the general formula I are promising as new fertility regulating agents without the usual side effects on the somatic cells which are known from the hitherto used hormonal contraceptives which are based on estrogens and/or gestagens.

For use as a contraceptive agent in females, a meiosis inducing substance can be administered so as to prematurely induce resumption of meiosis in oocytes while they are still in the growing follicle, before the ovulatory peak of gonadotropins occurs. In women, the resumption of the meiosis can, for example, be induced a week after the preceding menstruation has ocased. When ovulated, the resulting overmature oocytes are then most likely not to be fertilized. The normal menstrual cycle is not likely to be affected. In this connection it is important to notice, that the biosynthesis of progesterone in cultured human granulosa cells (somatic cells of the follicle) is not affected by the presence of a meiosis inducing substance whereas the estrogens and gestagens used in the hitherto used hormonal contraceptives do have an adverse effect on the biosynthesis of progesterone.

According to another aspect of this invention, a meiosis inducing substance of the general formula I can be used in the treatment of certain cases of infertility in females, including women, by administration thereof to females who, due to an insufficient own production of meiosis activating substance, are unable to produce mature oocytes. Also, when *in vitro* fertilization is performed, better results can be achieved, when a compound of the invention is added to the medium in which the oocytes are cultured.

When infertility in males, including men, is caused by an insufficient own production of the meiosis activating substance and thus a lack of mature sperm cells exists, administration of a compound of the invention may relieve the problem.
As an alternative to the method described above, contraception in females can also be achieved by administration of a compound of the invention which inhibits the meiosis, so that no mature oocytes are produced. Similarly, contraception in males can be achieved by administration of a compound of the invention which inhibits the meiosis, so that no mature sperm cells are produced.

The route of administration of compositions containing a compound of the invention may be any route which effectively transports the active compound to its site of action.

Thus, when the compounds of this invention are to be administered to a mammal, they are conveniently provided in the form of a pharmaceutical composition which comprises at least one compound of the invention in connection with a pharmaceutically acceptable carrier. For oral use, such compositions are preferably in the form of capsules or tablets.

From the above it will be understood that administrative regimen called for will depend on the condition to be treated. Thus, when used in the treatment of infertility the administration may have to take place once only, or for a limited period, e.g. until pregnancy is achieved.
When used as a contraceptive, the compounds of the invention will either have to be administered continuously or cyclically. When used as a contraceptive by females and not taken continuously, the timing of the administration relative to the ovulation will be important.

### The present invention is further illustrated by the following examples:

### Synthesis of the intermediate cholesta-4,8-dien-3-one

A solution of 2.20 g cholesta-5,8-dien-3β-ol in 27 ml toluene and 6 ml cyclohexanone is refluxed for 10 minutes in a Dean-Stark apparatus. 0.57 g aluminum isopropoxide are added and the reaction mixture is refluxed for 30 minutes. After cooling and addition of sulfuric acid (2 N), the resulting mixture is extracted with ethyl acetate. The organic layer is separated, washed with saturated sodium bicarbonate solution and water, dried over anhydrous sodium sulphate and filtered. After evaporation of the solvent the residue is chromatographed with a mixture of hexane and ethyl acetate to give 1.53 g cholesta-4,8-dien-3-one as a white solid.
¹H-NMR (CDCl₃): δ= 0.68 (s, 3H, H-18); 0.87 (2x d, J=7 Hz, 6H, H-26/27); 0.94 (d, J=7 Hz, 3H, H-21); 1.35 (s, 3H, H-19); 5.77 (s, 1H, H-4)

Cholesta-4,8(14)-dien-3-one is synthesized from cholesta-5,8(14)-dien-3β-ol in the same way. Cholesta-4,7-dien-3-one is synthesized according to literature procedures [*Just. Liebigs Ann. Chem.* **542**, 218, (1939)].

### Example 1 (scheme 1):

### Cholesta-4,8-dien-3α-ol and cholesta-4,8-dien-3β-ol

To a solution of 520 mg cholesta-4,8-dien-3-one in 45 ml tetrahydrofuran 1.70 ml of a L-Selectride solution (1 N, in tetrahydofuran) are added dropwise at -75 °C. The reaction mixture is warmed to room temperature within 4 hours, poured into hydrochloric acid (1 N) and extracted with ethyl acetate. The organic layer is separated, washed with brine, dried over anhydrous sodium sulphate and filtered. After evaporation of the solvent the residue is chromatographed with a mixture of hexane and ethyl acetate to give 286 mg cholesta-4,8-dien-3β-ol and 20 mg cholesta-4,8-dien-3α-ol as white solids.

### Cholesta-4,8-dien-3α-ol

¹H-NMR (CDCl₃): δ= 0.63 (s, 3H, H-18); 0.86 (2x d, J=7 Hz, 6H, H-26/27); 0.92 (d, J=7 Hz, 3H, H-21); 1.11 (s, 3H, H-19); 4.04 (m, 1H, H-3); 5.47 (d, H=5 Hz, 1H, H-4)

### Cholesta-4,8-dien-3β-ol

¹H-NMR (CDCl₃): δ= 0.64 (s, 3H, H-18); 0.86 (2x d, J=7 Hz, 6H, H-26/27); 0.93 (d, J=7 Hz, 3H, H-21); 1.23 (s, 3H, H-19); 2.47 (m, 1H); 4.19 (m, 1H, H-3); 5.32 (s, 1H, H-4)

Cholesta-4,7-dien-3α-ol and cholesta-4,7-dien-3β-ol are synthesized according to literature procedures [*Just. Liebigs Ann. Chem.* **542**, 218, (1939)].

### Example 2 (scheme 2):

### a) 5-cyano-5β-cholest-8-en-3-one and 5-cyano-5α-cholest-8-en-3-one

30 ml of a diethylaluminum cyanide solution (1 N, in toluene) are added to a solution of 3.82 g cholesta-4,8-dien-3-one in 60 ml tetrahydrofuran at 0°C. The reaction mixture is warmed to room temperature and stirred for 4 hours. 20 ml of a sodium hydroxide solution (1 N) are added before the mixture is extracted with diethyl ether. The organic layer is separated, dried over anhydrous sodium sulphate and filtered. After evaporation of the solvent the residue is chromatographed with a mixture of hexane and ethyl acetate to give 1.46 g 5-cyano-5β-cholest-8-en-3-one und 1.76 g 5-cyano-5α-cholest-8-en-3-one as pale yellow crystals.

### 5-cyano-5β-cholest-8-en-3-one:

¹H-NMR (CDCl₃): δ= 0.69 (s, 3H, H-18); 0.87 (2x d, J=7 Hz, 6H, H-26/27); 0.95 (d, J=7 Hz, 3H, H-21); 1.43 (s, 3H, H-19); 2.63 (m, 1H)

### 5-cyano-5α-cholest-8-en-3-one:

¹H-NMR (CDCl₃): δ= 0.64 (s, 3H, H-18); 0.87 (2x d, J=7 Hz, 6H, H-26/27); 0.95 (d, J=7 Hz, 3H, H-21); 1.37 (s, 3H, H-19); 2.52 (m, 2H)

5-cyano-5β-cholest-7-en-3-one [which is known from the literature: *Aust. J. Chem.* **35**, 629, (1982)], 5-cyano-5α-cholest-7-en-3-one, 5-cyano-5β-cholest-8(14)-en-3-one und 5-cyano-5α-cholest-8(14)-en-3-one are synthesized from the corresponding starting materials in the same way.

### b) 5-cyano-5α-cholest-8-en-3-β-ol and 5-cyano-5α-cholest-8-en-3-α-ol

327 mg sodium borohydride are added to a solution of 1.76 g 5-cyano-5α-cholest-8-en-3-one in 200 ml ethanol at room temperature. The reaction mixture is stirred for 4 hours. After addition of hydrochloric acid (1 N) the resulting mixture is extracted with dichloromethane. The organic layer is separated, dried over anhydrous sodium sulphate and filtered. After evaporation of the solvent the residue is chromatographed with a mixture of hexane and ethyl acetate to give 0.36 mg 5-cyano-5α-cholest-8-en-3-β-ol und 1.00 g 5-cyano-5α-cholest-8-en-3-α-ol as white solids.

### 5-cyano-5α-cholest-8-en-3-β-ol

¹H-NMR (CDCl₃): δ= 0.59 (s, 3H, H-18); 0.86 (2x d, J=7 Hz, 6H, H-26/27); 0.92 (d, J=7 Hz, 3H, H-21); 1.08 (s, 3H, H-19); 4.12 (bm, 1H, H-3)

### 5-cyano-5α-cholest-8-en-3-α-ol

¹H-NMR (CDCl₃): δ= 0.63 (s, 3H, H-18); 0.89 (2x d, J=7 Hz, 6H, H-26/27); 0.94 (d, J=7 Hz, 3H, H-21); 1.06 (s, 3H, H-19); 4.11 (bm, 1H, H-3)

5-cyano-5α-cholest-7-en-3β-ol, 5-cyano-5α-cholest-7-en-3α-ol, 5-cyano-5β-cholest-7-en-3α-ol, 5-cyano-5β-cholest-8-en-3β-ol, 5-cyano-5β-cholest-8-en-3α-ol, 5-cyano-5α-cholest-8(14)-en-3β-ol, 5-cyano-5α-cholest-8(14)-en-3α-ol, 5-cyano-5β-cholest-8(14)-en-3β-ol und 5-cyano-5β-cholest-8(14)-en-3α-ol are synthesized in the same way.

### Example 3 (scheme 3):

### a) 3β-hydroxy-5α-cholest-8-ene-5-carbaldehyde

4.66 ml of a diisobutylaluminum hydride solution (1.2 N, in toluene) are added to a solution of 230 mg 5-cyano-5α-cholest-8-en-3-β-ol in 18 ml toluene at - 10 °C. The reaction mixture is stirred for 3 hours before 3.6 ml sulfuric acid (1 N) are added. After refluxing for one hour the resulting mixture is cooled to room temperature, diluted with water and extracted with dichloromethane. The organic layer is separated, dried over anhydrous sodium sulphate and filtered. After evaporation of the solvent 220 mg 3β-hydroxy-5α-cholest-8-ene-5-carbaldehyde are obtained as a white solid.
¹H-NMR (CDCl₃): δ= 0.63 (s, 3H, H-18); 0.86 (2x d, J=7 Hz, 6H, H-26/27); 0.94 (d, J=7 Hz, 3H, H-21); 1.17 (s, 3H, H-19); 2.23 (m, 2H); 3.61 (m, 1H, H-3); 9.86 (s, 1 H, 5-CHO)

### b) 5-(hydroxymethyl)-5α-cholest-8-en-3β-ol

A suspension of 56 mg lithiumaluminum hydride in 5 ml tetrahydrofuran is added to a solution of 200 mg 3β-hydroxy-5α-cholest-8-en-5-carbaldehyde in 20 ml tetrahydrofuran at room temperature. After being heated to 50 °C for two hours the reaction mixture is cooled to room temperature. 0.06 ml water, 0.06 ml of a sodium hydroxide solution (1 N) and 0.18 ml water are added subsequently. The resulting suspension is stirred for 15 minutes and filtered over anhydrous sodium sulphate. After evaporation of the solvent the residue is crystallized from ethylacetate to give 80 mg 5-(hydroxymethyl)-5α-cholest-8-en-3β-ol as a white solid.
¹H-NMR (CDCl₃): δ= 0.63 (s, 3H, H-18); 0.87 (2x d, J=7 Hz, 6H, H-26/27); 0.93 (d, J=7 Hz, 3H, H-21); 1.16 (s, 3H, H-19); 3.58 (s, 2H 5-CH₂OH); 3.97 (m, 1H, H-3)

5-(hydroxymethyl)-5α-cholest-7-en-3β-ol, 5-(hydroxymethyl)-5β-cholest-8-en-3α-ol and 5-(hydroxymethyl)-5α-cholest-8(14)-en-3β-ol are synthesized in the same way.

### Example 4 (scheme 4):

### 3',4α-dihydrocyclopropa[4,5]-5β-cholest-8-en-3β-ol

300 mg zinc dust and 0.03 ml glacial acetic acid are added to a solution 5.4 mg cupric acetate in 1.2 ml dimetoxyethane at room temperature. The mixture is stirred for 30 minutes before 0.01 ml triethylamine are added. After 5 minutes a solution of 100 mg cholesta-4,8-dien-3β-ol in 0.4 ml dimethoxyethane is added. Then 0.24 ml diiodomethane are added at such a rate that the reaction temperature does not rise above 40 °C. The reaction mixture is stirred for further 6 hours. After addition of saturated ammonium chloride solution the resulting mixture is extracted with ethyl acetate. The organic layer is separated, washed with brine, dried over anhydrous sodium sulphate and filtered. After evaporation of the solvent the residue is chromatographed with a mixture of hexane and ethyl acetate to give 32 mg 3',4α-dihydrocyclopropa[4,5]-5β-cholest-8-en-3β-ol as a white solid.
¹H-NMR (CDCl₃): δ= 0.25 (dd, J=9 Hz, 5 Hz, 1H, 4,5-CH₂); 0.63 (s, 3H, H-18); 0.76 (dd, J=5 Hz, 5 Hz, 1H, 4,5-CH₂); 0.87 (2x d, J=7 Hz, 6H, H-26/27); 0.94 (d, J=7 Hz, 3H, H-21); 1.11 (s, 3H, H-19); 4.32 (m, 1H, H-3)

### Example 5 (scheme 5):

### a) 5-methyl-5β-cholest-8-en-3-one

0.85 ml of a methyllithium solution (1.6 N, in diethyl ether) are added to a suspension of 130 mg cuprous iodide in diethyl ether at 0 °C. The resulting mixture is stirred for one hour before a solution of 130 mg cholesta-4,8-dien-3-one in 1 ml diethyl ether is added. After being stirred for 30 minutes the reaction mixture is poured into saturated ammonium chloride solution and extracted with ethyl acetate. The organic layer is separated, washed with brine, dried over anhydrous sodium sulphate and filtered. After evaporation of the solvent the residue is chromatographed with a mixture of hexane and ethyl acetate to give 80 mg 5-methyl-5β-cholest-8-en-3-one as a white solid.
¹H-NMR (CDCl₃): δ= 0.63 (s, 3H, H-18); 0.86 (2x d, J=7 Hz, 6H, H-26/27); 0.90 (s, 3H, 5-CH₃); 0.93 (d, J=7 Hz, 3H, H-21); 1.06 (s, 3H, H-19); 2.42 (m, 1H)

### b) 5-methyl-5β-cholest-8-en-3α-ol und 5-methyl-5β-cholest-8-en-3β-ol

0.31 ml of a K-Selectride solution (1 N, in tetrahydrofuran) are added to a solution of 63 mg 5-methyl-5β-cholest-8-en-3-one in 4.4 ml tetrahydrofuran at - 65 °C. After being stirred for 2 hours the reaction mixture is allowed to warm to room temperature, poured into saturated ammonium chloride solution and extracted with ethyl acetate. The organic layer is separated, washed with brine, dried over anhydrous sodium sulphate and filtered. After evaporation of the solvent the residue is chromatographed with a mixture of dichloromethane and acetone to give 26 mg 5-methyl-5β-cholest-8-en-3α-ol and 21 mg 5-Methyl-5β-cholest-8-en-3β-ol as white solids.

### 5-methyl-5β-cholest-8-en-3α-ol

¹H-NMR (CDCl₃): δ= 0.59 (s, 3H, H-18); 0.85 (2x d, J=7 Hz, 6H, H-26/27); 0.86 (s, 3H); 0.90 (s, 3H); 0.92 (d, J=7 Hz, 3H, H-21); 3.82 (m, 1H, H-3)

### 5-methyl-5β-cholest-8-en-3β-ol

¹H-NMR (CDCl₃): δ= 0.63 (s, 3H, H-18); 0.87 (2x d, J=7 Hz, 6H, H-26/27); 0.89 (s, 3H, 5-CH₃); 0.93 (d, J=7 Hz, 3H, H-21); 0.99 (s, 3H, H-19), 3.87 (m, 1H, H-3)

5-methyl-5β-cholest-8(14)-en-3-one, 5-methyl-5β-cholest-8(14)-en-3β-ol and 5-methyl-5β-cholest-8(14)-en-3α-ol are synthesized in the same way.

### Example 6 (scheme 1):

### 3β-(trifluoromethyl)-cholesta-4,8-dien-3α-ol and 3α-(trifluoromethyl)-cholesta-4,8-dien-3β-ol

0.2 ml triethylsilyl-trifluoromethane and 328 mg tetrabutylammonium fluoride trihydrate are added to a solution of 200 mg cholesta-4,8-dien-3-one in 10 ml tetrahydrofuran at room temperature. After being stirred for 2 hours the reaction mixture is diluted with ethyl acetate, washed with water and brine, dried over anhydrous sodium sulphate and filtered. After evaporation of the solvent the residue is chromatographed with a mixture of hexane and ethyl acetate to give 45 mg 3β-(trifluoromethyl)-cholesta-4,8-dien-3α-ol as a colourless oil and 170 mg 3α-(trifluormethyl)-cholesta-4,8-dien-3β-ol as a white solid.

### 3β-(trifluoromethyl)-cholesta-4,8-dien-3α-ol:

¹H-NMR (CDCl₃): δ= 0.65 (s, 3H, H-18); 0.87 (2x d, J=7 Hz, 6H, H-26/27); 0.93 (d, J=7 Hz, 3H, H-21); 1.18 (s, 3H, H-19); 2.41 (m, 1H,); 5.39 (s, 1H, H-4)

### 3α-(trifluoromethyl)-cholesta-4,8-dien-3β-ol:

¹H-NMR (CDCl₃): δ= 0.64 (s, 3H, H-18); 0.87 (2x d, J=7 Hz, 6H, H-26/27); 0.93 (d, J=7 Hz, 3H, H-21); 1.25 (s, 3H, H-19); 2.43 (m, 1H,); 5.25 (s, 1H, H-4)

## Claims

1. Compounds of the general formula I 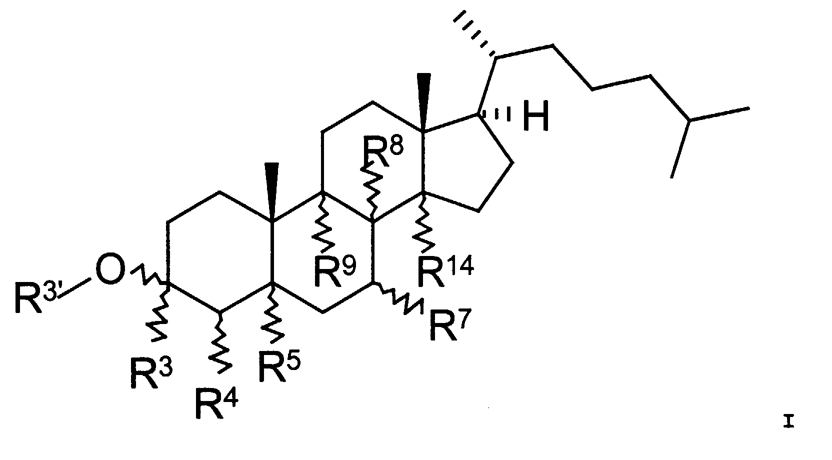 wherein
R³ designates a hydrogen atom, a C₁-C₆-alkyl group, a C₁-C₆-perfluoroalkyl group or together with R^{3'} an additional bond,
R^{3'} designates a hydrogen atom or together with R³ an additional bond,
R⁴ designates a hydrogen atom or together with R⁵ a methano bridge or together with R⁵ an additional bond,
R⁵ designates a C₁-C₆-alkyl group, a cyano group, a hydroxymethyl group, a carbaldehyde, an oxime derived from a carbaldehyde, a carboxylic acid, a primary or secondary amide derived from a carboxylic acid, an ester with a C₁-C₆-alcohol group or together with R⁴ a methano bridge or together with R⁴ an additional bond,
R⁷ designates together with R⁸ an additional bond or a hydrogen atom, if R⁸ and R⁹ or R⁸ and R¹⁴ stand together for an additional bond,
R⁸ designates together with R⁷ or with R⁹ or with R¹⁴ an additional bond,
R⁹ designates together with R⁸ an additional bond or a hydrogen atom, if R⁷ and R⁸ or R⁸ and R¹⁴ stand together for an additional bond,
R¹⁴ designates together with R⁸ an additional bond or a hydrogen atom, if R⁷ and R⁸ or R⁸ and R⁹ stand together for an additional bond,
or esters thereof,
with the proviso that the following compounds are disclaimed:
cholesta-4,7-dien-3-one, cholesta-4,8-dien-3-one, cholesta-4,8(14)-dien-3-one, cholesta-4,7-dien-3α-ol, cholesta-4,7-dien-3β-ol, 5-cyano-5β-cholest-7-en-3-one, 5-cyano-5β-cholest-7-en-3β-ol, 5-methyl-5β-cholest-7-en-3-one, 5-methyl-5β-cholest-7-en-3α-ol, 5-methyl-5β-cholest-7-en-3β-ol.

2. Compounds according to claim 1, wherein R³ designates a hydrogen atom, a C₁-C₆-perfluoroalkyl group, or together with R^{3'} an additional bond.

3. Compounds according to claim 1 or 2, wherein R³ designates a C₁-C₃-perfluoroalkyl group, preferably a trifluoromethyl group.

4. Compounds according to any one of claims 1 to 3, wherein R⁵ designates a C₁-C₃-alkyl group, preferably a methyl group, a cyano group, a hydroxymethyl group or together with R⁴ a methano bridge or together with R⁴ an additional bond.

5. Compounds according to any one of claims 1 to 4 which are
cholesta-4,8-dien-3β-ol,
cholesta-4,8-dien-3α-ol,
cholesta-4,8(14)-dien-3β-ol,
cholesta-4,8(14)-dien-3α-ol,
5-cyano-5α-cholest-7-en-3α-ol,
5-cyano-5α-cholest-7-en-3β-ol,
5-cyano-5β-cholest-7-en-3α-ol,
5-cyano-5α-cholest-8-en-3α-ol,
5-cyano-5α-cholest-8-en-3β-ol,
5-cyano-5β-cholest-8-en-3α-ol,
5-cyano-5β-cholest-8-en-3β-ol,
5-cyano-5α-cholest-8(14)-en-3α-ol,
5-cyano-5α-cholest-8(14)-en-3β-ol,
5-cyano-5β-cholest-8(14)-en-3α-ol,
5-cyano-5β-cholest-8(14)-en-3β-ol,
3',4α-dihydrocyclopropa[4,5]-5β-cholest-7-en-3β-ol,
3',4β-dihydrocyclopropa[4,5]-5α-cholest-7-en-3α-ol,
3',4α-dihydrocyclopropa[4,5]-5β-cholest-8-en-3β-ol,
3,4β-dihydrocyclopropa[4,5]-5α-cholest-8-en-3α-ol,
3',4α-dihydrocyclopropa[4,5]-5β-cholest-8(14)-en-3β-ol,
3',4β-dihydrocyclopropa[4,5]-5α-cholest-8(14)-en-3α-ol,
5-(hydroxymethyI)-5α-cholest-7-en-3β-ol,
5-(hydroxymethyl)-5β-cholest-7-en-3α-ol,
5-(hydroxymethyl)-5α-cholest-8-en-3β-ol,
5-(hydroxymethyl)-5β-cholest-8-en-3α-ol,
5-(hydroxymethyl)-5α-cholest-8(14)-en-3β-ol,
5-(hydroxymethyl)-5β-cholest-8(14)-en-3α-ol,
5-methyl-5β-cholest-8-en-3-one,
5-methyl-5β-cholest-8-en-3β-ol,
5-methyl-5β-cholest-8-en-3α-ol,
5-methyl-5β-cholest-8(14)-en-3-one,
5-methyl-5β-cholest-8(14)-en-3β-ol,
5-methyl-5β-cholest-8(14)-en-3α-ol,
3α-(trifluoromethyl)-cholesta-4,7-dien-3β-ol,
3β-(trifluoromethyl)-cholesta-4,7-dien-3α-ol,
3α-(trifluoromethyl)-cholesta-4,8-dien-3β-ol,
3β-(trifluoromethyl)-cholesta-4,8-dien-3α-ol,
3α-(trifluoromethyl)-cholesta-4,8(14)-dien-3β-ol,
3β-(trifluoromethyl)-cholesta-4,8(14)-dien-3α-ol.

6. Pharmaceutical compositions comprising one or more compounds of the general formula I according to any one of claims 1 to 5 as active substances.

7. Use of the compounds of the general formula I according to any one of claims 1 to 5 for the preparation of a meiosis regulating medicament.

8. Use according to claim 7 for the preparation of a medicament for the treatment of infertility in females or males, preferably in humans.

9. Use according to claim 7 for the preparation of a contraceptive medicament for the treatment of females or males, preferably humans.

10. Use of the compounds of the general formula I according to any one of claims 1 to 5 to regulate the fertilisation rate in fertilisation culture media.

11. A method of regulating meiosis comprising administering to a subject in need of such a regulation an effective amount of one or more compounds of the general formula I according to any one of claims 1 to 5.

12. A method of regulating meiosis in a mammalian germ cell comprising administering *ex vivo* or *in vitro* to a germ cell in need of such a regulation an effective amount of one or more compounds of the general formula I according to any one of claims 1 to 5.

13. A method according to claim 12 wherein the germ cell is an oocyte or a male germ cell.
